# EUROPEAN PATENT APPLICATION

(11) **EP 2 679 173 A1**
(43) Date of publication of application: **01.01.2014**
(21) Application number: 13173866.8
(22) Date of filing: 26.06.2013
(51) Int. Cl.: A61B 17/15

(54) **Cutting block including modular mounting systems**

(30) Priority: 30.06.2012 US 201213539358
(71) Applicant: DePuy (Ireland), Ringaskiddy (IE)
(72) Inventor: Edwards, Jon M, Warsaw, IN 46581 (US); Pagnano, Mark W, Rochester, MN 55906 (US)
(74) Representative: Belcher, Simon James

(57) **Abstract**

An orthopaedic surgical instrument (10) includes a cutting block (12) having a bone-engaging surface (24) and an outer surface (22) positioned opposite the bone-engaging surface, a first cutting guide slot (40) defined in the cutting block, and a second cutting guide slot (70, 72) defined in the cutting block. The orthopaedic surgical instrument includes two mounting systems (14, 16) configured to separately secure the cutting block to a bone of a patient.

## Description

The present invention relates to orthopaedic surgical instruments and, more particularly, to surgical instruments used to resect a patient's bone.

Joint arthroplasty is a well-known surgical procedure by which a diseased and/or damaged natural joint is replaced by a prosthetic joint. Typical artificial joints include knee prostheses, hip prostheses, shoulder prostheses, ankle prostheses, and wrist prostheses, among others. To facilitate the replacement of the natural joint with the prosthesis, orthopaedic surgeons use a variety of orthopaedic surgical instruments such as, for example, saws, drills, reamers, rasps, broaches, cutting blocks, drill guides, milling guides, and other surgical instruments.

The invention provides an orthopaedic surgical instrument which includes a cutting block having a bone-engaging surface and an outer surface positioned opposite the bone-engaging surface, a first cutting guide slot defined in the cutting block, and a second cutting guide slot defined in the cutting block. The first cutting guide slot defines a first imaginary plane, the second cutting guide slot defines a second imaginary plane that extends oblique to the first imaginary plane. The orthopaedic surgical instrument includes a first mounting system configured to secure the cutting block to a bone of a patient. The first mounting system includes a plate removably coupled to the cutting block, and a pair of spikes attached to the plate, each spike having a bone-engaging tip. The orthopaedic surgical instrument also includes a second mounting system configured to secure the cutting block to the bone of the patient. The second mounting system includes a plurality of guide bores extending through the bone-engaging surface and the outer surface of the cutting block, and a plurality of fixation pins configured to be received in the plurality of guide bores, each fixation pin having a bone-engaging tip.

Optionally, the second mounting system may be configured to secure the cutting block to the bone of the patient in place of the first mounting system when the plate is detached from the cutting block. Optionally, the orthopaedic surgical instrument may include a third cutting guide slot defined in the cutting block. The second cutting guide slot is positioned between the first cutting guide slot and the third cutting guide slot.

Optionally, the plurality of guide bores may be positioned between the first cutting guide slot and the second cutting guide slot. Optionally, the plurality of guide bores may be a first plurality of guide bores, and the second mounting system may include a second plurality of guide bores that are defined in the cutting block between the second cutting guide slot and the third cutting guide slot.

Optionally, the orthopaedic surgical instrument may include a first guide bore positioned on a first side of the second cutting guide slot and a second guide bore positioned on a second side of the second cutting guide slot. Each of the first guide bore and the second guide bore may be sized to receive a fixation pin.

Optionally, the bone-engaging surface of the cutting block may have an aperture defined therein, and the plate of the first mounting system may be positioned in the aperture. Optionally, the orthopaedic surgical instrument may include a second plate configured to be positioned in the aperture defined in the bone-engaging surface. The second plate may be devoid of spikes. Optionally, the pair of spikes and the plate may be formed as a single monolithic component.

The invention also provides an orthopaedic surgical instrument which includes a cutting block having a bone-engaging surface including an aperture defined therein, a first bracket positioned in the aperture defined in the cutting block, and a second bracket configured to be positioned in the aperture of the cutting block in place of the first bracket. The second bracket has a pair of spikes extending outwardly therefrom, and each spike having a bone-engaging tip. The first bracket and the cutting block cooperate to define a cutting guide slot, and the second bracket and the cutting block cooperate to define the cutting guide slot when the second bracket is positioned in the aperture of the cutting block.

Optionally, the cutting guide slot may be a first cutting guide slot that defines a first imaginary plane, and the cutting block may have a second cutting guide slot defined therein. The second cutting guide slot may define a second imaginary plane that extends oblique to the first imaginary plane.

Optionally, the first cutting guide slot may be a femoral chamfer cutting guide slot and the second cutting guide slot may be an anterior cutting guide slot. Optionally, the orthopaedic surgical instrument may include a first mounting system including the second bracket, and a second mounting system configured to secure the cutting block to a bone of a patient in place of the first mounting system. The second mounting system may include a plurality of guide bores defined in the cutting block, and a plurality of fixation pins configured to be received in the plurality of guide bores. Each fixation pin may have a bone-engaging tip.

Optionally, the plurality of guide bores may be positioned between the femoral chamfer cutting guide slot and the anterior cutting guide slot. Optionally, the orthopaedic surgical instrument may further include a posterior cutting guide slot defined in the cutting block.

Optionally, the plurality of guide bores may be a first plurality of guide bores. The second mounting system may include a second plurality of guide bores that are defined in the cutting block between the posterior cutting guide slot and the femoral chamfer cutting guide slot.

Optionally, the first bracket and the cutting block may cooperate to define a third cutting guide slot, and the second bracket and the cutting block may cooperate to define the third cutting guide slot when the second bracket is positioned in the aperture of the cutting block.

The invention also provides an orthopaedic surgical instrument which includes a cutting block, an anterior cutting guide slot defined in the cutting block, an anterior chamfer cutting guide slot defined in the cutting block, and a first mounting system configured to secure the cutting block to a bone of a patient. The first mounting system includes a plate removably coupled to the cutting block and a pair of spikes attached to the plate, each spike having a bone-engaging tip. The orthopaedic surgical instrument also includes a second mounting system configured to secure the cutting block to the bone of the patient when the plate is detached from the cutting block. The second mounting system includes a plurality of guide bores extending through the cutting block, and a plurality of fixation pins configured to be received in the plurality of guide bores. Each fixation pin has a bone-engaging tip.

Optionally, the cutting block may include a body having an aperture defined therein. The plate may be received in the aperture.

Optionally, the plate and the body may cooperate to define the anterior chamfer cutting guide slot.

Optionally, the orthopaedic surgical instrument may include a posterior chamfer cutting guide slot defined by the plate and the body of the cutting block.

Optionally, the orthopaedic surgical instrument may include a second plate configured to be positioned in the aperture in place of the plate. The second plate may cooperate to define the anterior chamfer cutting guide slot and the posterior chamfer cutting guide slot when the second plate is positioned in the aperture.

The invention is described below by way of example with reference to the accompanying drawings, in which:
FIG. 1 is an exploded perspective anterior view of an orthopaedic surgical instrument.
FIG. 2 is an exploded perspective posterior view of the orthopaedic surgical instrument of FIG. 1.
FIG. 3 is a perspective view of a pair of fixation pins attached to a distal end of a patient's femur.
FIG. 4 is a plan view of the orthopaedic surgical instrument of FIG. 1 positioned on the fixation pins of FIG. 3.
FIG. 5 is a perspective view similar to FIG. 3 showing the orthopaedic surgical instrument positioned on the distal end of the patient's femur.
FIG. 6 is a perspective view of the distal end of the patient's femur and the orthopaedic surgical instrument of FIG. 1.
FIG. 7 is an exploded perspective view of another orthopaedic surgical instrument.

Terms representing anatomical references, such as anterior, posterior, medial, lateral, superior and inferior are used in this document to refer to the orthopaedic implants and surgical instruments described herein as well as in reference to the patient's natural anatomy. Such terms have well-understood meanings in both the study of anatomy and the field of orthopaedics. Use of such anatomical reference terms in this document is intended to be consistent with their well-understood meanings unless noted otherwise.

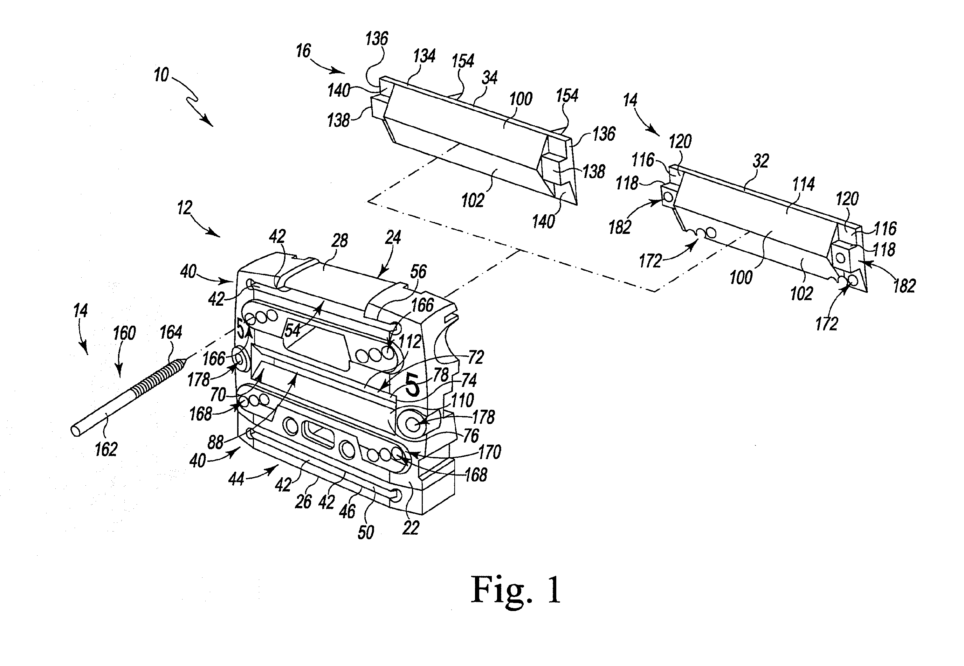

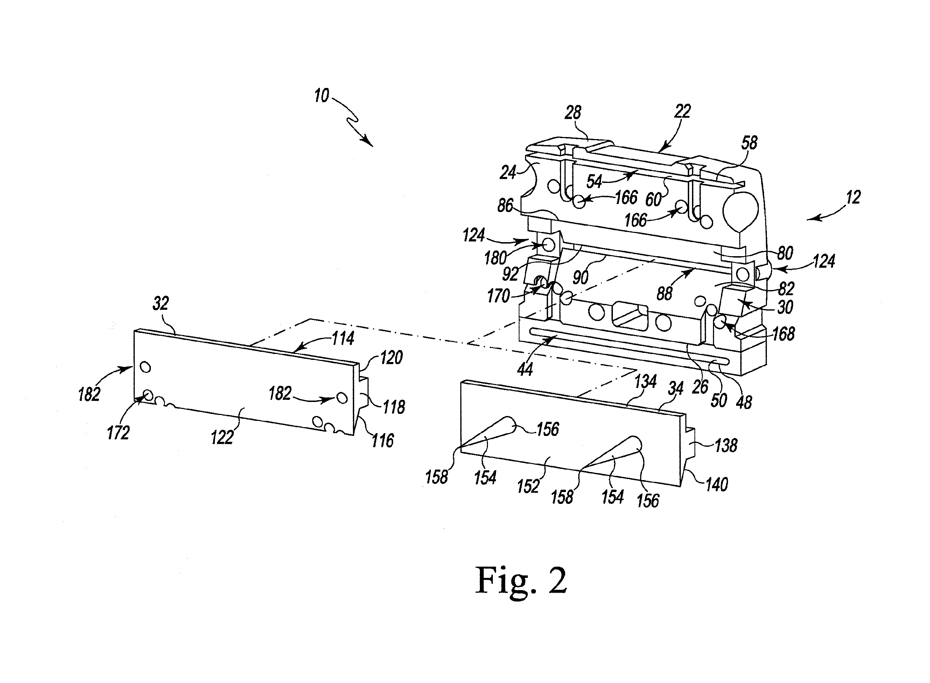

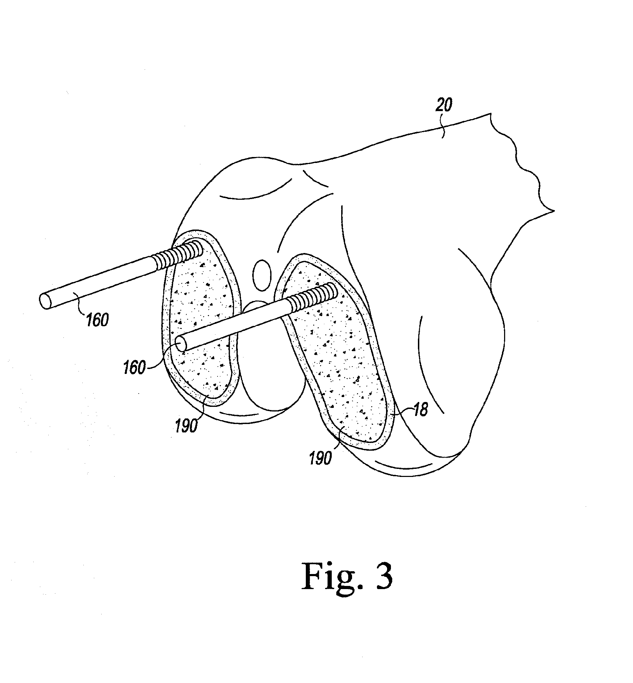

Referring to the drawings, FIGS. 1 and 2 show an orthopaedic surgical instrument 10 which includes a cutting block 12 and a pair of modular mounting systems 14, 16 that are configured to secure the cutting block 12 to a distal end 18 of a patient's femur 20 (see FIG. 3). The cutting block 12 includes an outer surface 22 and a bone-engaging surface 24 positioned opposite the outer surface 22. The surfaces 22, 24 of the cutting block 12 extend from a lower end 26 to an upper end 28. As shown in FIG. 2, the surface 24 of the cutting block 12 has an aperture 30 defined therein.

The modular mounting system 14 includes a plate or bracket 32 that is sized and shaped to be positioned in the aperture 30 of the cutting block 12. The other modular mounting system 16 includes a plate or bracket 34 that is sized and shaped to be positioned in the aperture 30 in place of the bracket 32 of the system 14. The surgical instrument 10 also includes a plurality of cutting slots or guides 40, and each cutting guide 40 is defined by opposing guide surfaces 42, as described in greater detail below. Each cutting guide 40 is sized and shaped to receive the blade (not shown) of a surgical saw or other cutting instrument and orient the blade to resect the patient's bone during an orthopaedic surgical procedure.

In the device shown in the drawings, the cutting block 12 and the brackets 32, 34 of the instrument 10 are formed from metallic materials such as, for example, steel, titanium alloy, or cobalt chromium alloy. It should be appreciated that the cutting block 12 and the brackets 32, 34 may be formed from polymeric materials such as, for example, polyamide, poly(phenylsulphone), or polyketone. The guide surfaces 42 of the instrument 10 may then be formed from a metallic material such as those described above. It should be understood that various combinations of polymeric and metallic materials may be used to form the cutting block 12 and the brackets 32, 34.

As shown in FIG. 1, a posterior cutting guide 44 of the cutting guides 40 is defined at the lower end 26 of the cutting block 12. The cutting guide 44 is defined between opposing guide surfaces 42, which extend between an opening 46 defined in the outer surface 22 and another opening 48 (see FIG. 2) defined in the bone-engaging surface 24. An imaginary resection plane 50 is defined by the cutting guide 44. The resection plane 50 extends through the distal end 18 of the patient's femur 20 when the cutting block 12 is secured thereto. In that way, the posterior cutting guide 44 may be used by the surgeon during the resection of the patient's femur 20, as described in greater detail below.

The cutting guides 40 of the instrument 10 also include an anterior cutting guide 54, which is defined at the upper end 28 of the cutting block 12. The cutting guide 54 is defined between opposing guide surfaces 42, which extend between an opening 56 (see FIG. 1) defined in the outer surface 22 and another opening 58 (see FIG. 2) defined in the bone-engaging surface 24. An imaginary resection plane 60 is defined by the cutting guide 44. The resection plane 60 extends through the distal end 18 of the patient's femur 20 when the cutting block 12 is secured thereto. In that way, the anterior cutting guide 54, like the posterior cutting guide 44, may be used by the surgeon during the resection of the patient's femur 20.

The orthopaedic surgical instrument 10 also includes a pair of chamfer cutting guides 70, 72 positioned between the posterior cutting guide 44 and the anterior cutting guide 54. In the device shown in the drawings, the cutting block 12 and the brackets 32, 34 cooperate to define the chamfer cutting guides 70, 72. As shown in FIG. 1, the cutting block 12 has an opening 74 defined in the outer surface 22, and planar guide surfaces 76, 78 extend inwardly from the opening 74. As shown in FIG. 2, a pair of planar guide surfaces 80, 82 extend inwardly from an opening 86 defined in the bone-engaging surface 24. The guide surfaces 76, 82 of the cutting block 12 are connected at an inner edge 90, and the guide surfaces 78, 80 are connected at an inner edge 92. As shown in FIGS. 1 and 2, a slot 88 is defined between the inner edges 90, 92, and the surfaces 80, 82 cooperate to define the aperture 30 in the bone-engaging surface 24.

As shown in FIG. 1, each of the brackets 32, 34 includes a planar upper guide surface 100 and a planar lower guide surface 102. When the bracket 32 is positioned in the aperture 30 of the cutting block 12, the upper guide surface 100 of the bracket 32 faces the guide surface 80 of the cutting block 12 such the upper guide surface 100 cooperates with the guide surfaces 76, 80 to define the chamfer cutting guide 70. The lower guide surface 102 of the bracket 32 faces the guide surface 82 of the cutting block 12 such that the lower guide surface 102 cooperates with the guide surfaces 78, 82 to define the other chamfer cutting guide 72.

Similarly, when the bracket 34 is positioned in the aperture 30 in place of the bracket 32, the upper guide surface 100 of the bracket 34 faces the guide surface 80 of the cutting block 12 such the upper guide surface 100 cooperates with the guide surfaces 76, 80 to define the chamfer cutting guide 70. The lower guide surface 102 of the bracket 34 faces the guide surface 82 of the cutting block 12 such that the lower guide surface 102 cooperates with the guide surfaces 78, 82 to define the other chamfer cutting guide 72.

An imaginary resection plane 110 is defined by the chamfer cutting guide 70. The resection plane 110 extends through the distal end 18 of the patient's femur 20 when the cutting block 12 is secured thereto. The other chamfer cutting guide 72 defines another imaginary resection plane 112, which also extends through the distal end 18 of the patient's femur 20 when the cutting block 12 is secured thereto. In that way, the chamfer cutting guides 70, 72 may be used by the surgeon during the resection of the patient's femur 20. The resection planes 110, 112 of the chamfer cutting guides 70, 72 extend obliquely relative to the resection planes 50, 60 defined by the posterior cutting guide 44 and the anterior cutting guide 54, respectively.

As shown in FIGS. 1 and 2, the bracket 32 of the modular mounting system 14 includes a body 114 and a pair of flanges 116 extending outwardly from the body 114. In the device shown in the drawings, the body 114 is triangular and includes the upper guide surface 100 and the lower guide surface 102. Each flange 116 has a plug 118 extending from an inner surface 120. The body 114 and the flanges 116 cooperate to define a bone-engaging surface 122 of the bracket 32.

When the bracket 32 is secured to the cutting block 12, the bone-engaging surface 122 is co-planar with the bone-engaging surface 24 of the cutting block 12. Additionally, as shown in FIG. 2, the aperture 30 defined in the cutting block 12 includes a pair of notches 124 positioned on each side of the slot 88. When the bracket 32 is secured to the cutting block 12, each plug 118 of the bracket 32 is received in a corresponding notch 124 of the cutting block 12.

The orthopaedic surgical instrument 10 includes a locking mechanism configured to secure the bracket 32 to the cutting block 12. In the device shown in the drawings, the locking mechanism includes the plugs 118 of the brackets 32 and the surfaces defining the notches 124 of the cutting block 12. The plugs 118 are sized to engage the surfaces defining the notches 124, thereby creating a frictional interlock between them and securing the bracket 32 to the cutting block 12. The instrument 10 may include different locking mechanisms such as, for example, fasteners, latches, tabs, and so forth to secure the bracket 32 to the cutting block 12.

As shown in FIGS. 1 and 2, the bracket 34 of the modular mounting system 16 includes a body 134 and a pair of flanges 136 extending outwardly from the body 134. In the device shown in the drawings, the body 134 is triangular and includes the upper guide surface 100 and the planar guide surface 102. Each flange 136 has a plug 138 extending from an inner surface 140. The body 134 and the flanges 136 cooperate to define a bone-engaging surface 152 of the bracket 34.

The bracket 34 of the modular mounting system 16 also includes a pair of spikes 154 that extend outwardly from the bone-engaging surface 152. As shown in FIG. 2, each spike 154 has a base 156 secured to the bone-engaging surface 152 and a bone-engaging tip 158. In the device shown in the drawings, the spikes 154, the body 134, and the flanges 136 are formed as a single monolithic component.

When the bracket 34 is secured to the cutting block 12, the bone-engaging surface 152 is co-planar with the bone-engaging surface 24 of the cutting block 12, and the spikes 154 extend outwardly therefrom. Additionally, each plug 138 of the bracket 34 is received in a corresponding notch 124 of the cutting block 12 when the bracket 34 is secured to the cutting block 12. Similar to the bracket 34, the plugs 138 of the bracket 34 and the surfaces of the notches 124 of the cutting block 12 form a locking mechanism configured to secure the bracket 34 to the cutting block 12. The plugs 138 are sized to engage the surfaces defining the notches 124, thereby creating a frictional interlock between them and securing the bracket 34 to the cutting block 12.

The modular mounting system 14 of the instrument 10 also includes a plurality of fixation pins 160. As shown in FIG. 1, each fixation pin 160 includes a cylindrical shaft 162 having a threaded tip 164. The cutting block 12 has a plurality of guide bores 166 defined therein that are sized to receive the fixation pins 160. The bores 166 are positioned between the anterior cutting guide 54 and the opening 74 of the chamfer cutting guides 70, 72 and extend between the outer surface 22 and the bone-engaging surface 24 of the cutting block 12. The bores 166 are arranged in a staggered pattern to permit the surgeon to change the position of the cutting block 12 on the patient's femur 20 without having to remove the fixation pins 160, as described in greater detail below.

The modular mounting system 14 also includes another plurality of guide bores 168 positioned between the opening 74 of the chamfer cutting guides 70, 72 and the posterior cutting guide 44. Each guide bore 168 is sized to receive one of the fixation pins 160. As shown in FIGS. 1 and 2, each guide bore 168 includes a through-hole 170 extending between the outer surface 22 and the bone-engaging surface 24 of the cutting block 12 and another through-hole 172 extending through the bracket 32. In that way, when the bracket 32 is attached to the cutting block 12, the through-holes 172 of the bracket 32 cooperate with the through-holes 170 of cutting block 12 to define the guide bores 168. The bores 168 are arranged in a staggered pattern to permit the surgeon to change the position of the cutting block 12 on the patient's femur 20 without having to remove the fixation pins 160, as described in greater detail below.

The modular mounting system 14 of the orthopaedic surgical instrument 10 further includes a guide bore 178 positioned on each side of the opening 74 of the chamfer cutting guides 70, 72. Each guide bore 178 is sized to receive one of the fixation pins 160. As shown in FIGS. 1 and 2, each guide bore 178 includes a through-hole 180 extending between the outer surface 22 and the bone-engaging surface 24 of the cutting block 12 and another through-hole 182 extending through the bracket 32. In that way, when the bracket 32 is attached to the cutting block 12, the through-holes 182 of the bracket 32 cooperate with the through-holes 180 of cutting block 12 to define the guide bores 178.

In operation, the surgeon may utilize the orthopaedic surgical instrument 10 to prepare the distal end 18 of the patient's femur 20 to receive a prosthetic femoral component. To do so, the surgeon may secure the cutting block 12 to the patient's femur 20 using the modular mounting system 14 or the modular mounting system 16. The surgeon may then use the cutting guides 40 of the cutting block 12 to guide a cutting saw blade in making a series of resections of the distal end 18 of the patient's femur 20.

During an orthopaedic surgical procedure, the surgeon may first resect the distal end 18 of the patient's femur 20 to create a surgically-prepared distal surface 190. If the surgeon selects the modular mounting system 14, the instrument 10 may be assembled by aligning the bracket 32 with the aperture 30 defined in the cutting block 12. The surgeon or other user may advance the bracket 32 into the aperture 30 such that the plugs 118 of the bracket 32 are received in the notches 124 of the cutting block 12. As described above, the plugs 118 frictionally engage the cutting block 12 to secure the bracket 32 to the cutting block 12.

The surgeon may secure a pair of fixation pins 160 of the modular mounting system 14 to the surgically-prepared distal surface 190 of the patient's femur 20, as shown in FIG. 3. To do so, the surgeon may size the patient's femur 20 for the prosthetic femoral component and set the femoral rotation. A suitable procedure for locating fixation pins during a femoral sizing and rotation setting procedure is described in the surgical technique document that is available from DePuy Orthopaedics Inc relating to the instrument that is identified by the trade mark SIGMA Fixed. After sizing the femoral component and setting the rotation, the surgeon may attach the fixation pins 160 to the surgically-prepared distal surface 190 of the patient's femur 20.

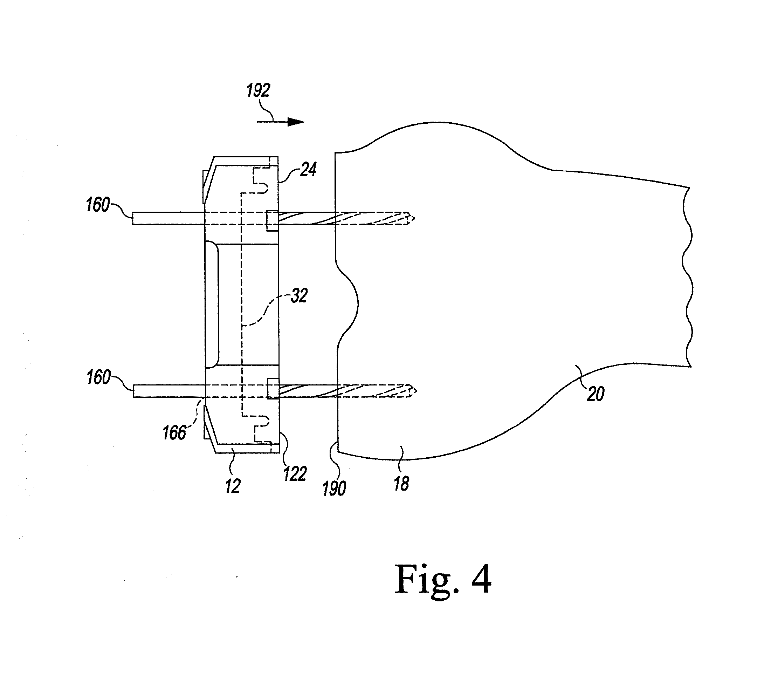

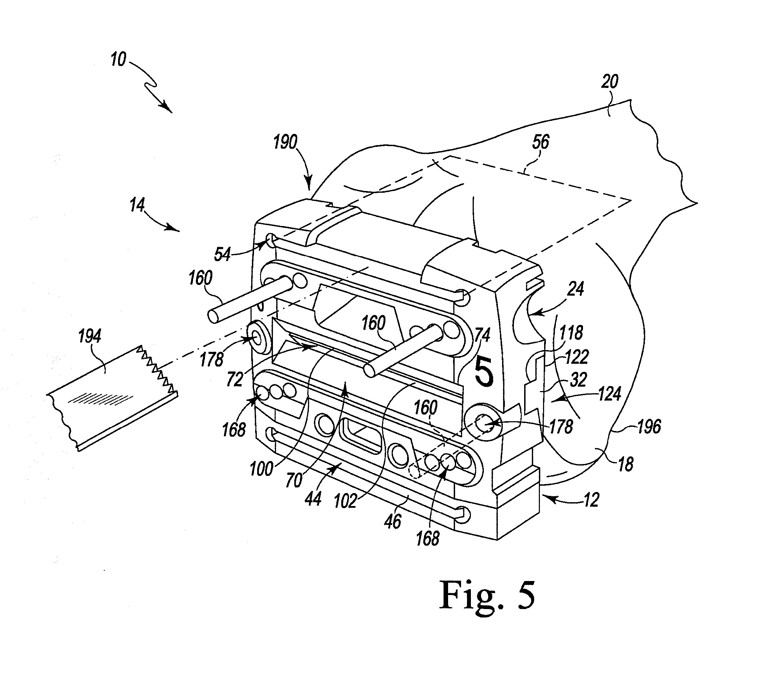

After attaching the fixation pins 160, the surgeon may position the cutting block 12 and the bracket 32 on the surgically-prepared distal surface 190. To do so, the surgeon may align the shafts 162 of the fixation pins 160 with a pair of guide bores 166 of the cutting block 12. The surgeon may then advance the cutting block 12 over the shafts 162 in the direction indicated by arrow 192 in FIG. 4. The bone-engaging surface 122 of the bracket 32 and the bone-engaging surface 24 of the cutting block 12 contact the surgically-prepared distal surface 190 when the instrument 10 is positioned on the distal end 18 of the patient's femur 20, as shown in FIG. 5. If the surgeon desires to relocate the cutting block 12, the surgeon may utilize another combination of guide bores 166 to change the position of the cutting block 12 on the patient's femur 20. If additional fixation is necessary, the surgeon may insert additional fixation pins 160 through the bores 168 defined in the cutting block 12.

The surgeon may use the cutting block 12 to make a number of resections of the distal end 18 of the patient's femur 20. For example, as shown in FIG. 5, the anterior cutting guide 54 defines a resection plane 56 that extends through the distal end 18 of the patient's femur 20. The surgeon may advance a cutting tool, such as, for example, a surgical cutting saw 194 through the cutting guide 54 to engage the patient's femur 20 and operate the surgical saw 194 to surgically prepare an anterior surface of the patient's femur 20 to receive the prosthetic femoral component. The surgeon may similarly use the posterior cutting guide 44 to resect the posterior condyles 196 of the patient's femur 20 and surgically prepare the posterior surfaces of the patient's femur 20 to receive the prosthetic femoral component.

The surgeon may also use the chamfer cutting guides 70, 72 of the instrument 10 to make chamfer cuts on the patient's femur 20. To do so, the surgeon may insert fixation pins 160 through the bores 178 positioned on each side of the opening 74 of the chamfer cutting guides 70, 72. The surgeon may remove any fixation pins 160 from the bores 166, 168 before advancing the surgical cutting saw 194 through each chamfer cutting guides 70, 72 to resect the distal end 18 of the patients' femur 20.

Instead of the modular mounting system 14, the surgeon may select the modular mounting system 16 to secure the cutting block 12 to the patient's femur 20. In that case, the instrument 10 may be assembled by aligning the bracket 34 with the aperture 30 defined in the cutting block 12. The surgeon or other user may advance the bracket 34 into the aperture 30 such that the plugs 138 of the bracket 34 are received in the notches 124 of the cutting block 12. As described above, the plugs 138 frictionally engage the cutting block 12 to secure the bracket 34 to the cutting block 12.

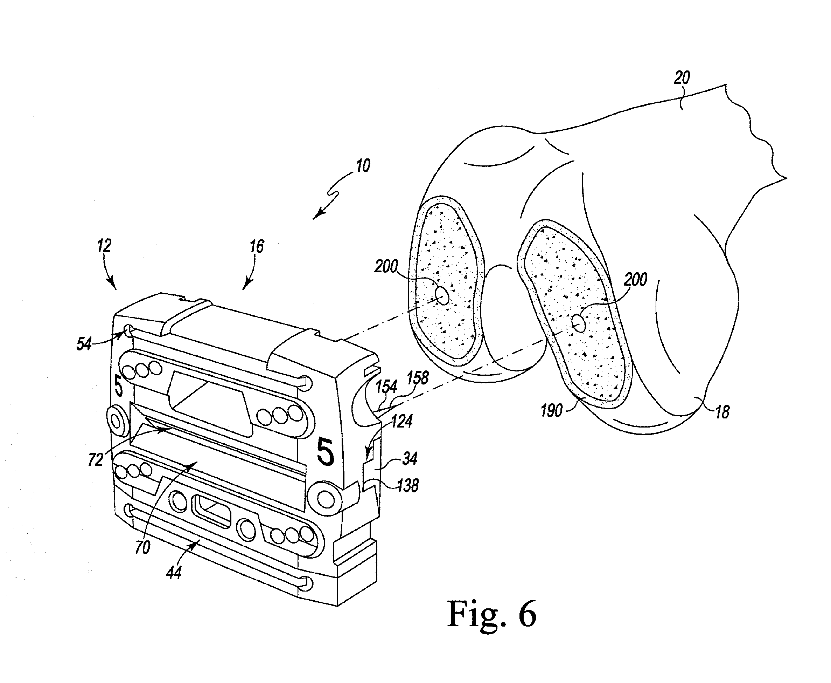

The surgeon may form a pair of holes 200 in the surgically-prepared distal surface 190 of the patient's femur 20. As shown in FIG. 6, each hole 200 is sized to receive a corresponding spike 154 of the modular mounting system 16. To locate the holes, the surgeon may size the patient's femur 20 for the prosthetic femoral component and set the femoral rotation. One exemplary procedure for locating the holes during a femoral sizing and rotation setting procedure is described in the surgical technique document that is available from DePuy Orthopaedics Inc relating to the instrument that is identified by the trade mark SIGMA Classic. After sizing the femoral component and setting the rotation, the surgeon may form the holes 200 in the surgically-prepared distal surface 190 of the patient's femur 20.

After forming the holes 200, the surgeon may position the cutting block 12 and the bracket 34 on the surgically-prepared distal surface 190. To do so, the surgeon may align the spikes 154 of the modular mounting system 16 with the holes 200 and advance the tips 158 of the spikes 154 into the holes 200. The bone-engaging surface 152 of the bracket 34 and the bone-engaging surface 24 of the cutting block 12 contact the surgically-prepared distal surface 190 when the instrument 10 is positioned on the distal end 18 of the patient's femur 20.

As described above in regard to the modular mounting system 14, the surgeon may use the cutting block 12 to make a number of resections of the distal end 18 of the patient's femur 20. The surgeon may advance the surgical cutting saw 194 through the cutting guide 54 to engage the patient's femur 20 and operate the surgical saw 194 to surgically prepare an anterior surface of the patient's femur 20 to receive the prosthetic femoral component. The surgeon may similarly use the posterior cutting guide 44 to resect the posterior condyles 196 of the patient's femur 20 and surgically prepare the posterior surfaces of the patient's femur 20 to receive the prosthetic femoral component. The surgeon may also use the chamfer cutting guides 70, 72 of the instrument 10 to make chamfer cuts on the patient's femur 20.

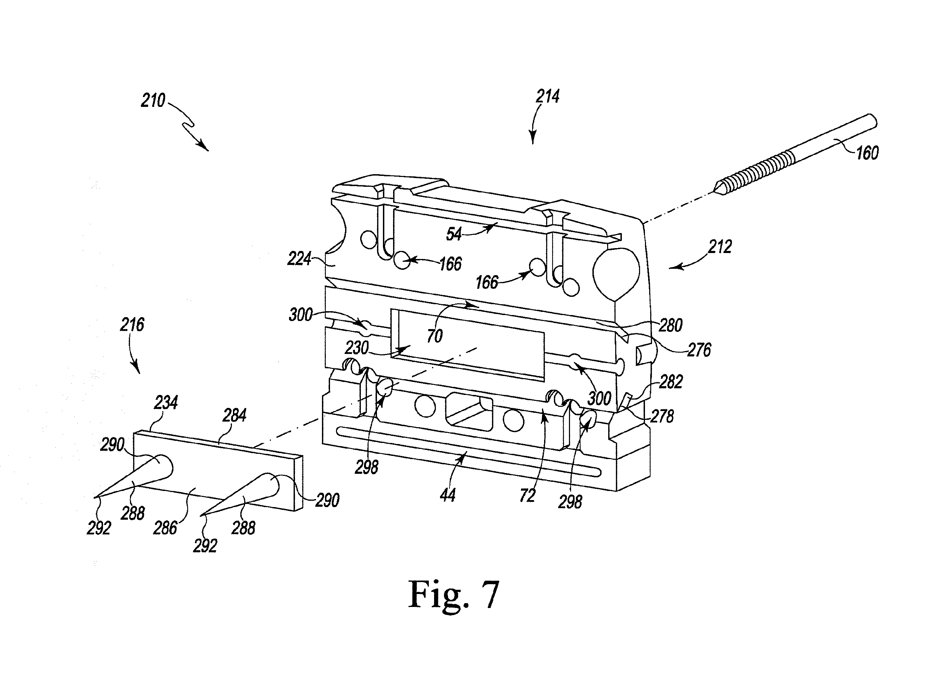

FIG. 7 shows another orthopaedic surgical instrument (hereinafter instrument 210) in which some features are substantially similar to those of the instrument described above with reference to FIGS. 1 to 6. Such features are designated in FIG. 7 with the same reference numbers as those used in FIGS. 1 to 6. Like the instrument 10 described above with reference to FIGS. 1 to 6, the instrument 210 includes a cutting block 212 and a pair of modular mounting systems 214, 216 that are configured to secure the cutting block 212 to the distal end 18 of the patient's femur 20. The cutting block 212 includes an outer surface 22 and a bone-engaging surface 224 positioned opposite the outer surface 22. The surfaces 22, 224 of the cutting block 212 extend from a lower end 26 to an upper end 28. As shown in FIG. 2, the surface 224 of the cutting block 212 has an aperture 230 defined therein.

The modular mounting system 216 includes a plate or bracket 234 that is sized and shaped to be positioned in the aperture 230. The surgical instrument 10 also includes a plurality of cutting slots or guides 40, and each cutting guide 40 is defined by opposing guide surfaces 42. Each cutting guide 40 is sized and shaped to receive the blade (not shown) of a surgical saw or other cutting instrument and orient the blade to resect the patient's bone during an orthopaedic surgical procedure.

The instrument 210 includes a posterior cutting guide 44, which is defined at the lower end 26 of the cutting block 212, and an anterior cutting guide 54, which is defined at the upper end 28 of the cutting block 212. The instrument 210 also includes a pair of chamfer cutting guides 270, 272 positioned between the posterior cutting guide 44 and the anterior cutting guide 54. Similar to the cutting block 12 described above with reference to FIGS. 1 to 6, the cutting block 212 has an opening (not shown) defined in the outer surface 22. A pair of opposing guide surfaces 276, 280 cooperate to define the chamfer cutting guide 270 in the cutting block 212. Another pair of opposing guide surfaces 278, 282 cooperate to define the chamfer cutting guide 272 in the cutting block 212.

As shown in FIG. 7, the bracket 234 of the modular mounting system 16 includes a body 284 sized and shaped to be received in the aperture 230 defined in the cutting block 212. The bracket 234 of the modular mounting system 16 also includes a bone-engaging surface 286 and a pair of spikes 288 that extend outwardly from the bone-engaging surface 286. Each spike 288 has a base 290 secured to the bone-engaging surface 286 and a bone-engaging tip 292. In the device shown in the drawings, the spikes 288 and the body 284 are formed as a single monolithic component.

When the bracket 234 is secured to the cutting block 212, the bone-engaging surface 286 is co-planar with the bone-engaging surface 224 of the cutting block 212, and the spikes 288 extend outwardly therefrom. The body 284 is sized to engage the surfaces defining the aperture 230, thereby creating a frictional interlock between them and securing the bracket 234 to the cutting block 212. In other constructions, the instrument 210 may include other locking mechanisms such as, for example, fasteners, latches, tabs, and so forth to secure the bracket 234 to the cutting block 212.

The modular mounting system 214, like the modular mounting system 14 described above with reference to FIGS. 1 to 6, includes a plurality of fixation pins 160 and a plurality of guide bores 166, 298, 300 defined in the cutting block 212 that are sized to receive the fixation pins 160. The bores 166, 298 are arranged in a staggered pattern to permit the surgeon to change the position of the cutting block 12 on the patient's femur 20 without having to remove the fixation pins 160.

In operation, the surgeon may use the orthopaedic surgical instrument 210 to prepare the distal end 18 of the patient's femur 20 to receive a prosthetic femoral component. To do so, the surgeon may secure the cutting block 212 to the patient's femur 20 using the modular mounting system 214 or the modular mounting system 216. The surgeon may then use the cutting guides 40 of the cutting block 212 to guide a cutting saw blade in making a series of resections of the distal end 18 of the patient's femur 20.

If the surgeon selects the modular mounting system 214, the surgeon may secure a pair of fixation pins 160 of the modular mounting system 214 to the surgically-prepared distal surface 190 of the patient's femur 20 in the manner described above with reference to FIGS. 1 to 6. After attaching the fixation pins 160, the surgeon may position the cutting block 212 on the surgically-prepared distal surface 190 and use the cutting block 12 to make a number of resections of the distal end 18 of the patient's femur 20.

Instead of the modular mounting system 214, the surgeon may select the modular mounting system 216 to secure the cutting block 212 to the patient's femur 20. In that case, the instrument 210 may be assembled by aligning the bracket 234 with the aperture 230 defined in the cutting block 212. The surgeon or other user may advance the bracket 234 into the aperture 230 such that the body 284 of the bracket 234 is received in the aperture 230 of the cutting block 212. As described above, the body 284 frictionally engages the cutting block 212 to secure the bracket 234 thereto.

The surgeon may form a pair of holes 200 in the surgically-prepared distal surface 190 of the patient's femur 20 in the manner described above in regard to FIGS. 1 to 6. After forming the holes 200, the surgeon may position the cutting block 212 and the bracket 234 on the surgically-prepared distal surface 190. To do so, the surgeon may align the spikes 288 of the modular mounting system 216 with the holes 200 and advance the tips 292 of the spikes 288 into the holes 200. The bone-engaging surface 286 of the bracket 234 and the bone-engaging surface 224 of the cutting block 212 contact the surgically-prepared distal surface 190 when the instrument 10 is positioned on the distal end 18 of the patient's femur 20. The surgeon may then use the cutting block 212 to make a number of resections of the distal end 18 of the patient's femur 20.

## Claims

1. An orthopaedic surgical instrument comprising:
a cutting block having a bone-engaging surface and an outer surface positioned opposite the bone-engaging surface,
a first cutting guide slot defined in the cutting block, the first cutting guide slot defining a first imaginary plane,
a second cutting guide slot defined in the cutting block, the second cutting guide slot defining a second imaginary plane that extends oblique to the first imaginary plane,
a first mounting system configured to secure the cutting block to a bone of a patient, the first mounting system including (i) a plate removably coupled to the cutting block, and (ii) a pair of spikes attached to the plate, each spike having a bone-engaging tip, and
a second mounting system configured to secure the cutting block to the bone of the patient, the second mounting system including (i) a plurality of guide bores extending through the bone-engaging surface and the outer surface of the cutting block, and (ii) a plurality of fixation pins configured to be received in the plurality of guide bores, each fixation pin having a bone-engaging tip.

2. The orthopaedic surgical instrument of claim 1, in which the second mounting system is configured to secure the cutting block to the bone of the patient in place of the first mounting system when the plate is detached from the cutting block.

3. The orthopaedic surgical instrument of claim 1, which includes a third cutting guide slot defined in the cutting block, in which the second cutting guide slot is positioned between the first cutting guide slot and the third cutting guide slot.

4. The orthopaedic surgical instrument of claim 3, in which the plurality of guide bores are positioned between the first cutting guide slot and the second cutting guide slot, and which the second mounting system preferably includes a second plurality of guide bores that are defined in the cutting block between the second cutting guide slot and the third cutting guide slot.

5. The orthopaedic surgical instrument of claim 1, which includes a first guide bore positioned on a first side of the second cutting guide slot and a second guide bore positioned on a second side of the second cutting guide slot, each of the first guide bore and the second guide bore being sized to receive a fixation pin.

6. The orthopaedic surgical instrument of claim 1, in which the bone-engaging surface of the cutting block has an aperture defined therein, and the plate of the first mounting system is positioned in the aperture, and in which the instrument preferably includes a second plate configured to be positioned in the aperture defined in the bone-engaging surface, the second plate being devoid of spikes.

7. An orthopaedic surgical instrument comprising:
a cutting block including a bone-engaging surface having an aperture defined therein,
a first bracket positioned in the aperture defined in the cutting block, and
a second bracket configured to be positioned in the aperture of the cutting block in place of the first bracket, the second bracket having a pair of spikes extending outwardly therefrom, each spike having a bone-engaging tip,
in which (i) the first bracket and the cutting block cooperate to define a cutting guide slot, and (ii) the second bracket and the cutting block cooperate to define the cutting guide slot when the second bracket is positioned in the aperture of the cutting block.

8. The orthopaedic surgical instrument of claim 7, in which the cutting guide slot is a first cutting guide slot that defines a first imaginary plane, and the cutting block has a second cutting guide slot defined therein, the second cutting guide slot defining a second imaginary plane that extends oblique to the first imaginary plane.

9. The orthopaedic surgical instrument of claim 8, in which the first cutting guide slot is a femoral chamfer cutting guide slot and the second cutting guide slot is an anterior cutting guide slot.

10. The orthopaedic surgical instrument of claim 9, which includes a first mounting system including the second bracket, and a second mounting system configured to secure the cutting block to a bone of a patient in place of the first mounting system, the second mounting system including (i) a plurality of guide bores defined in the cutting block, and (ii) a plurality of fixation pins configured to be received in the plurality of guide bores, each fixation pin having a bone-engaging tip.

11. The orthopaedic surgical instrument of claim 10, in which the plurality of guide bores are positioned between the femoral chamfer cutting guide slot and the anterior cutting guide slot.

12. The orthopaedic surgical instrument of claim 11, which includes a posterior cutting guide slot defined in the cutting block, and in which the second mounting system includes a second plurality of guide bores that are defined in the cutting block between the posterior cutting guide slot and the femoral chamfer cutting guide slot.

13. The orthopaedic surgical instrument of claim 8, in which (i) the first bracket and the cutting block cooperate to define a third cutting guide slot, and (ii) the second bracket and the cutting block cooperate to define the third cutting guide slot when the second bracket is positioned in the aperture of the cutting block.

14. An orthopaedic surgical instrument comprising:
a cutting block,
an anterior cutting guide slot defined in the cutting block,
an anterior chamfer cutting guide slot defined in the cutting block,
a first mounting system configured to secure the cutting block to a bone of a patient, the first mounting system including (i) a plate removably coupled to the cutting block, and (ii) a pair of spikes attached to the plate, each spike having a bone-engaging tip, and
a second mounting system configured to secure the cutting block to the bone of the patient when the plate is detached from the cutting block, the second mounting system including (i) a plurality of guide bores extending through the cutting block, and (ii) a plurality of fixation pins configured to be received in the plurality of guide bores, each fixation pin having a bone-engaging tip.

15. The orthopaedic surgical instrument of claim 14, in which the cutting block includes a body having an aperture defined therein, and the plate is received in the aperture, and in which preferably the plate and the body cooperate to define the anterior chamfer cutting guide slot.
